# EUROPÄISCHE PATENTSCHRIFT

(11) **EP 1 114 006 B1**
(45) Veröffentlichungstag und Bekanntmachung des Hinweises auf die Patenterteilung: **09.10.2002**
(21) Anmeldenummer: 99938322.7
(22) Anmeldetag: 22.07.1999
(51) Int. Cl.: C04B 28/28, A61K 6/083, A61L 24/12

(54) **SPHÄRISCHE IONOMERPARTIKEL UND DEREN HERSTELLUNG**
SPHERICAL IONOMER PARTICLES AND PRODUCTION THEREOF
PARTICULES SPHERIQUES D'IONOMERE ET LEUR FABRICATION

(30) Priorität: 22.07.1998 DE 19832965
(43) Veröffentlichungstag der Anmeldung: 11.07.2001
(73) Patentinhaber: Fraunhofer-Gesellschaft zur Förderung der angewandten Forschung e.V., 80636 München (DE)
(72) Erfinder: WOLTER, Herbert, D-97950 Gerchsheim (DE); GELLERMANN, Carsten, D-97074 Würzburg (DE)
(86) Internationale Anmeldenummer: EP9905257
(87) Internationale Veröffentlichungsnummer: WO00005182

(56) Entgegenhaltungen:
- US-A- 4 215 033

## Beschreibung

Die vorliegende Erfindung betrifft anorganische bzw. ggf. organisch modifizierte Partikel, die gezielt einer Auslaugung bestimmter Kationen unterworfen werden können und damit für den Einsatz als anorganische Komponente in sogenannten Glasionomerzementen verwendbar sind. Des weiteren betrifft die Erfindung neuartige Wege der Herstellung solcher "lonomere" genannter Partikel sowie die damit herstellbaren Zemente.

Unter dem Ausdruck "lonomerpartikel" versteht man anorganische Teilchen, die in Verbindung mit einer bevorzugt säurehaltigen Matrix in sehr vielfältiger Weise als Zemente (selbsthärtbar, lichthärtbar usw.) eingesetzt werden können. Damit eine Zementbildungsreaktion überhaupt stattfinden kann, müssen diese Teilchen definiert bzw. gezielt instabil sein, d.h., sie müssen in Verbindung mit Wasser in Gegenwart des Partners, mit dem sie sich verbinden sollen, Metallionen abgeben, die zu einer Härtungsreaktion in der Partnersubstanz führen. Die Zusammensetzungsbereiche, in denen diese gezielte Instabilität auftritt, sind dem Fachmann bekannt bzw. ohne weiteres zu ermitteln, siehe z.B. die Phasendiagramme einer Vielzahl von Systemen in "Alumosilicate Glasses for Polyelectrolyte Cements", A.D. Wilson et al., Ind. Eng. Chem. Prod. Res. Dev. 1980, (19) 263-270, oder "Glass-lonomer Cements", A.D. Wilson et al., 1988, Quintessence Publishing Co., Inc., Chapter 2 (S. 21 ff.).

Der Grundaufbau der üblicherweise glasartigen lonomere besteht meist aus dem ternären System Siliciumdioxid-Aluminiumoxid-Calciumoxid. Durch Zusammenschmelzen dieser Komponenten erhält man Partikel, die in Gegenwart von z.B. Polyalkensäuren eine zweistufige Reaktion eingehen. Dabei werden durch die Attacke von Protonen der Polyalkensäuren zuerst Calciumionen aus dem Glasverbund herausgelöst, die in einer instabilen Phase oder sogenannten primären Härtung von den Carboxylatgruppen der Polyalkensäuren komplexiert werden. Die sekundäre Härtung führt sodann zu einer stabilen Phase, in der nun auch Aluminium-Kationen aus dem Glas-lonomer herauswandern. Unter Hydratation der Polysalze entstehen dabei Aluminium-Polyalkenoate. Gleichzeitig wird die äußere Schale der Aluminium-Silikatgläser durch den Protonenangriff unter Bildung von Orthokieselsäure aufgelöst. Diese Orthokieselsäure kondensiert im weiteren Verlauf der Reaktion zu Kieselgel; es resultiert eine Gelschicht.

Die beschriebenen Reaktionen sind zum Teil sehr langsam; die Reaktion läßt sich jedoch durch Zugabe von Fluorid, beispielsweise in Form von Flußspat, beschleunigen.

Hydroxycarbonsäuren, z.B. Weinsäure, können als Regulatoren zugesetzt werden; sie verlängern die Verarbeitungszeit und kürzen die Härtungszeit. Weitere Zusatzstoffe wie Aluminiumphosphat, Kryolit oder Aluminiumtrifluorid sind als optimierende Verarbeitungshilfen bekannt.

Die bekannten Ionomerpartikel werden durch Zusammenschmelzen der jeweiligen Ausgangsverbindungen (hauptsächlich von Oxiden) gewonnen. Ihr Gefüge ist komplex. Der Mahlprozeß, dem das zusammengeschmolzene Glas-lonomer unterworfen wird, um die erwünschten Partikel zu erhalten, fördert das Entstehen scharfkantiger, unrunder Teilchen. Dadurch ist die resultierende Abriebfestigkeit des Ionomerzustandes ungenügend. Die gebildeten Teilchen sind heterodispers und relativ groß; sie haben meist einen Durchmesser von weit über drei Mikrometern. Sie müssen einem aufwendigen Klassifizierungsprozeß unterworfen werden, um in einer auch nur einigermaßen akzeptablen Größenverteilung gewonnen werden zu können. Neben hohem Arbeitsaufwand bedeutet dies einen hohen Substanzverlust und damit extrem schlechte Ausbeuten (selbst bei Anwendung einer Mehrzahl von Siebungen oder Windsichtungen wird - schon aufgrund der ungünstigen Geometrie - eine Verteilung über weniger als mindestens eine Zehnerpotenz nicht erreicht). Die Aluminium-Silikat-Matrix ist infolge des Zusammenschmelzens häufig nicht homogen. So erfolgt z.B. die Einlagerung von Fluoriden in Form von caliciumfluoridreichen Tröpfchen.

Klassische Glasionomerzemente mit rein anorganischer Härtung, lichthärtende Glasionomerzemente (mit zusätzlichen organischen polymerisierbaren Komponenten) und sogenannte Kompomere (der Begriff leitet sich aus der Zusammenziehung der Ausdrücke Komposit und lonomer ab und soll solche Zemente benennen, in denen die Carboxylgruppe an demselben Molekül gebunden eingesetzt wird, das auch eine vernetzbare Doppelbindung trägt, siehe z.B. "Glasionomers, The next Generation", Proc. of the 2nd Int. Symp. on Glass Ionomers, 1994, S. 13 ff.) werden häufig als Füllmaterial insbesondere in der Zahnmedizin eingesetzt. Als Partner für die Härtung (Zementbildung) dienen dabei meist die genannten Polyalkensäuren. Vorteile dieser Materialien sind: kaum oder keine Schrumpfung bis hin zu einer durch die lonomerreaktion als Folge der Wasseraufnahme bedingten Expansion, Vorhandensein von Fluoriden und Phosphaten erwünscht, guter Verbund mit dem Zahngewebe bedingt durch die Säuregruppen in der Matrix. Allerdings wäre für den Einsatz in der Zahnmedizin auch eine ausgezeichnete Mechanik, ein günstiges Abriebverhalten (z.B. beim Kauen) und eine gute Polierbarkeit der Füllungen erforderlich. Diesen Anforderungen genügen die genannten Zemente jedoch infolge der Größe und Form der Ionomerpartikel nicht. So wirkt die Scharfkantigkeit, die Größe und Asymmetrie der Teilchen, daß sie in oberflächennahen Positionen beim Kauen oder Polieren aus dem Zementverbund gerissen werden, was den Abrieb verstärkt und ein Glätten kaum möglich macht. Zusätzliche Nachteile liegen in der mangelnden Anpassungsfähigkeit an spezielle Probleme wie Röntgenopazität oder spezielle Anforderungen an Transparenz wie z.B. den Brechungsindex oder dgl..

Aufgabe der vorliegenden Erfindung ist es daher, Ionomerpartikel der eingangs erwähnten Art bereitzustellen, die die oben erwähnten Nachteile nicht aufweisen und im Verbund mit beliebigen, bevorzugt säuremodifizierten Matrixsystemen zu mechanisch stabileren Zementen führen als die bekannten Partikel.

Die Aufgabe wird dadurch gelöst, daß lonomerpartikel zur Verfügung gestellt werden, die eine sphärische oder annähernd sphärische Form aufweisen.

Die lonomerpartikel sind entweder rein anorganische Partikel; sie können aber auch organisch modifiziert sein.

Bevorzugt besitzen die neuen lonomerpartikel einen geringeren Durchmesser als die derzeit üblichen. Die Partikelgröße kann auf z.B. 5 nm bis 50 µm eingestellt werden. Hierzu können verschiedene, einfache Verfahren gezielt eingesetzt werden, was weiter unten genauer erläutert wird.

Die sphärischen lonomerpartikel der vorliegenden Erfindung weisen einen inneren Bereich sowie einen Siliciumionen enthaltenden äußeren Bereich auf, dessen Kationen (a) mindestens ein Element, das in silikatischen Verbindungen Gitterplätze des Siliciums unter Entstehen eines negativen Ladungsüberschusses einnehmen kann, und (b) mindestens ein Element, das den negativen Ladungsüberschuß kompensieren kann und ausgewählt ist unter Elementen der ersten und zweiten Hauptgruppe sowie solchen, die in zweiwertiger Form auftreten können, umfassen. Die Kationen der Gruppe (b) dienen der primären Härtung in der instabilen Phase, die der Gruppe (a) der sekundären Härtung und dem Entstehen der stabilen Phase.

Die Ausdrücke "Siliciumionen" und "Kationen" sollen dabei ausdrücken, daß die betreffenden Elemente in gebundener Form vorliegen. Dies soll nicht ausschließen, daß sie in Strukturen mit mehr oder weniger kovalenten Anteilen eingebunden sind.

Die Partikel können geeignete Zusätze enthalten, beispielsweise Phosphat (z.B. als Aloder Ca-Phosphat) oder Fluorid (z.B. als NaF, CaF₂ oder AlF₃).

In bevorzugter Weise sind in den sphärischen lonomerpartikeln clusterartige silikathaltige Bereiche enthalten. In einer weiteren bevorzugten Ausgestaltung der Erfindung sind die Ionomerpartikel durchgehend homogen. In einer dritten bevorzugten Ausgestaltung bestehen sie aus einem inneren Bereich oder Kern, der von der Zusammensetzung des äußeren Bereichs (der "Schale") abweichen kann, wobei die Schale aus einer oder mehreren Schichten bestehen kann. Zwingend ist dabei aber in jedem Fall die oben erwähnte Zusammensetzung im äußeren Bereich, also in mindestens der äußersten Schicht. Wird der Aufbau mit einem vom äußeren Bereich verschiedenen inneren Bereich gewählt, so kann dieser ggf. inert gegen Auslaugung sein und als Träger für weitere Eigenschaften dienen.

Zusätzlich kann die Oberfläche der Partikel silanisiert sein, was die Einarbeitung in manche Matrices erleichtert. Die Silanisierung kann nach bekannten Verfahren erfolgen, je nach angewandter Methode in situ oder nachträglich.

Die Partikel können vollständig ausgefüllt (dicht) sein, ggf. aber auch eine poröse Struktur besitzen.

Die Partikelgröße kann je nach Herstellungsbedingungen schwanken; sie ist ggf. in einem engen Bereich einstellbar. Bevorzugt ist die Bereitstellung relativ kleiner Teilchen, da diese mehr Oberfläche aufweisen. Hierdurch wird die Reaktivität erhöht und damit die Härtung des Zementes beschleunigt oder verbessert. Ein weiterer Vorteil kleinerer Teilchen ist eine verbesserte Transluzenz des entstandenen Zementes. Beispiele für Teilchengrößen sind z.B. 20 nm bis 20 µm oder 0,5 µm bis 50 µm; die jeweils gewählte Teilchengröße läßt sich dabei in einem engen Verteilungssektor realisieren, der deutlich unter einer Zehnerpotenz liegen kann. Kleinere Teilchen, z.B. im Bereich von 50 nm bis 1 oder 2 µm, sind besonders gut für Zahnfüllungen geeignet. Neben den bereits beschriebenen Vorteilen läßt sich bei kleinen Teilchen auch ein besonders hoher Anteil von lonomer einarbeiten. Um einen ganz besonders hohen Anteil an lonomer im Zement erhalten zu können, wird in einer speziellen Ausgestaltung der Erfindung eine Mischung aus zwei oder drei Partien lonomerteilchen bereitgestellt, die in ihrer jeweils definiert engen Größenverteilung ein solches Größenverhältnis zueinander aufweisen, daß die kleineren Teilchen in die Lücken einer gedacht dichtesten Kugelpackung der größeren Teilchen passen und die ggf. vorhandenen, noch sehr viel kleineren Teilchen in Lücken der dabei entstehenden Packung hineinpassen. Auch diese Ausgestaltung ist für Zahnfüllungen besonders geeignet, weil ein hoher lonomeranteil im Zement realisiert werden kann. Neben relativ kleinen sollen jedoch erfindungsgemäß auch größere Teilchen zur Verfügung gestellt werden, sei es als größte Charge einer Größenmischung, wie voranstehend beschrieben, sei es für den Einsatz der Zemente auf anderen medizinischen oder außermedizinischen Feldern (z.B. als Knochensubstanz oder als Kleber).

Die Herstellung der sphärischen lonomerpartikel gelingt auf dem Weg über verschiedene Verfahren. Dabei wird eine organische Komponenten enthaltende Dispersion gebildet, in der eine kontrollierte Hydrolyse und Kondensation abläuft. Der Ausdruck "Dispersion" wird hier verwendet, obwohl möglicherweise auch echte Lösungen, Suspensionen oder Emulsionen erhalten werden können bzw. in bestimmten Stadien der hydrolytischen Kondensation entstehen können. Auch Sol- und Gelbildungsprozesse sollen von dem Ausdruck mit umfaßt sein (z.B. kann die disperse Phase einer Dispersion oder Emulsion gelieren). Er ist daher entsprechend breit zu verstehen. Die Dispersion kann auf verschiedenen Wegen, z.B. über den sog. Stöberprozeß oder eine Sprühtrocknung, in sphärische Partikel überführt werden. Als organische Komponente wird dabei mindestens eine Verbindung eingesetzt, die ausgewählt ist unter siliciumorganischen Verbindungen und organischen Verbindungen der Kationen der unter (a) und (b) genannten Elemente. Unter dem Ausdruck "organische Verbindung" soll dabei jede "metallorganische" Verbindung verstanden werden, die mindestens einen entweder über Sauerstoff an das Metall gebundenen oder komplexierten organischen Bestandteil oder einen derart an das Metall gebundenen organischen Bestandteil aufweist, daß in Gegenwart von Wasser, wäßrigen oder anderen Lösungsmitteln bzw. Dispersionsmitteln (z.B. Alkoholen) eine zumindest teilweise Hydrolyse dieser Verbindung einsetzen kann, die ggf. auch erst unter der Einwirkung von Säure oder Base anspringt, worauf die Verbindung einer kontrollierten Kondensation unterliegt, so daß sich im "Lösungsmittel" Ketten- oder Netzwerk-Kondensate ausbilden, jedoch keine unkontrollierten Fällungsreaktionen eintreten (der Ausdruck "Lösungsmittel" ist dabei natürlich so zu verstehen, daß das Mittel in der Regel keine echte Lösung der organischen Verbindung(en) bewirken wird, es entsteht meist eine Suspension, eine Dispersion, eine Emulsion, ein Sol oder ein Gel). Beispiele für organische Verbindungen sind z.B. Oxokomplexe, wie Alkoholate oder Carboxylate, aber auch andere geeignete Metallkomplexe oder metallorganische Verbindungen. Je nach Bedarf können auch mehrere oder alle Kationen der späteren sphärischen Partikel in Form der genannten organischen Verbindungen eingesetzt werden.

Bei den unter (a) einsetzbaren Elementen handelt es sich bevorzugt um solche der dritten Hauptgruppe, mit Bor, Aluminium, Gallium, Indium und Thallium, Auch Scandium, Yttrium und seltene Erden wie Lanthan, Cer, Gadolinium, Ytterbium kommen in Betracht. Aluminium ist wegen der günstigen Strukturen der Alumosilikate bevorzugt. Durch die Auswahl spezieller Elemente, z.B. sehr schwerer Elemente, lassen sich bestimmte Eigenschaften wie Röntgenopazität erzeugen.

Wenn das oder eines (oder alle) der Elemente, die unter (a) genannt sind, als organische Komponente eingesetzt werden soll, werden hierfür bevorzugt Oxokomplexe eingesetzt. Als Oxokomplexe eignen sich beispielsweise Alkoholate, Diketonate und Carboxylate. Als Beispiele für Alkoholate seien Ethanolat, sekundärund tertiär-Butylat, z.B. des Aluminiums, genannt. Als Carboxylat sei beispielhaft das der Oxalsäure oder Methacrylsäure genannt. Auch Acetate bzw. Acetylacetonate sowie weitere Komplexe mit Chelatbildnern kommen in Frage. Wenn statt dessen oder zusätzlich ein oder das (oder alle) unter (a) genannte(n) Element(e) nicht als organische Komponente eingesetzt werden soll, bietet sich der Einsatz in Form von im gewählten Lösungsmittel löslichen oder unlöslichen, ggf. äußerst feinen Pulvern der entsprechenden anorganischen Verbindungen, z.B. der Oxide, Halogenide (Chloride, Fluoride), Phosphate oder anderer Salze (z.B. AlCl₃), an.

Die Metalle, unter denen die Kationen der unter (b) genannten Gruppe ausgewählt werden können, umfassen z.B. Lithium, Natrium, Kalium, Rubidium, Cäsium, Beryllium, Magnesium, Calcium, Strontium und Barium, Zinn oder Zink (letztere in ihrer zweiwertigen Form). Durch die Auswahl der geeigneten Kationen lassen sich dabei spezifische Eigenschaften gezielt erzeugen, beispielsweise Röntgenopazität, Reaktivität, optische Eigenschaften oder dergleichen.

Wenn das oder eines (oder alle) der in der Gruppe (b) genannten Elemente als organische Komponente eingesetzt werden soll, kommen hierfür nicht nur, aber insbesondere die Carboxylate und Alkoholate in Betracht. Besonders bevorzugt sind Magnesium-, Calcium- und Strontiumacetat und die Alkoholate, z.B. Isopropanolate, dieser Elemente. Wenn statt dessen oder zusätzlich ein oder das (oder alle) unter (b) genannte(n) Element(e) nicht als organische Komponente eingesetzt werden soll, bietet sich der Einsatz in Form von im gewählten Lösungsmittel löslichen oder unlöslichen, ggf. äußerst feinen Pulvern der entsprechenden anorganischen Verbindungen, z.B. der Oxide, Halogenide (Chloride, Fluoride), Phosphate oder anderer Salze (z.B. MgCl₂, SnCl₂) an.

Die für den äußeren Bereich erforderlichen Siliciumionen können auf verschiedene Art und Weise in die lonomerpartikel eingearbeitet werden. So können der Dispersion beispielsweise hydrolysierbare Silane oder Siloxane zugesetzt werden, beispielsweise Alkyl- und/oder Alkoxysilane. In diesem Falle gelangt man zu Partikeln mit einer homogenen silikathaltigen Matrix im äußeren Bereich. Alternativ kann einer Dispersion mit Verbindungen der in Gruppe (a) und/oder in Gruppe (b) genannten, komplexierten Elemente z.B. eine zweite Dispersion aus Siliciumdioxid-Partikeln mit sehr geringem Durchmesser zugemischt werden. In diesem Falle bildet das Siliciumdioxid clusterartige Strukturen innerhalb des äußeren Bereiches der sich bildenden Partikel, die aufgrund ihres geringen Durchmessers sehr gut mit den Oxiden der anderen Elemente vernetzt sind.

Der oben erwähnten Dispersion können je nach Anwendungsbereich verschiedene weitere Substanzen zugemischt werden. Ein Beispiel ist die Einarbeitung von Zinndioxid-Partikeln in ein die vorgenannten Bestandteile enthaltendes Sol. Damit lassen sich sphärische Partikel mit einem inneren Bereich aus Zinndioxid erhalten, die beispielsweise eine gute Röntgenabsorption gewährleisten. Der Kern der lonomerpartikel kann statt dessen auch aus Siliciumdioxid bestehen; hierfür werden Siliciumdioxidpartikel der geeigneten Größe (z.B. mit einem Durchmesser von 30-100 nm (z.B. für den Dentalbereich) oder von 1 bis 2 µm) mit der Dispersion in Kontakt gebracht, so daß sich diese im äußeren Bereich um den Kern aus Siliciumdioxid legen kann. Die vorgenannten ionomerreaktiven Modifikationen sind nur beispielhaft genannt; es lassen sich alle möglichen Varianten ausführen, solange die lonomerpartikel in ihrem äußeren Bereich die vorgenannten ionomer reaktiven Bestandteile aufweisen.

Die vorerwähnten organisch modifizierten Bestandteile zur Erzeugung der Dispersion können beispielsweise in Wasser eingebracht und ggf. mit Essig oder Eisessig versetzt werden (oder in das bereits angesäuerte Lösungsmittel gegeben werden). Auch basische Lösungsmittel sind möglich. Alternativ können die organisch modifizierten Komponenten beispielsweise in einem nichtwäßrigen Dipersionsmittel, z.B. einem Alkohol, in geeigneter Weise mit einer für die erforderlichen Hydrolyseprozesse ausreichenden Menge an Wasser und ggf. Base oder Säure als Katalysator versetzt werden. Zuvor oder danach können die ggf. mitzuverarbeitenden anorganischen Substanzen eingearbeitet werden, die ggf. zuvor gelöst oder dispergiert werden. In dieser Umgebung setzt jeweils eine hydrolytische Kondensation der organisch modifizierten Bestandteile ein, wobei jedoch anhand der Reaktionsbedingungen darauf zu achten ist, daß Hydroxide oder Oxide nicht unkontrolliert ausfallen. Vielmehr erfolgt die Umwandlung in Ketten und/oder ein Netzwerk, in dem die vorhandenen Wasserstoffbrückenbindungen ausreichen, um ein stabiles Gerüst über den räumlichen Bereich hinweg, aus dem sich die Partikel entwickeln (also eine Dispersion oder Suspension) oder durch die gesamte Flüssigkeit (unter Bildung eines Sols oder Gels) zu erhalten.

Aus den vorstehend beschriebenen Bestandteilen entsteht eine Dispersion, die erfindungsgemäß in sphärische oder annähernd sphärische Partikel überführt wird, bzw. aus der solche Partikel abgetrennt werden. Dies kann auf verschiedenen Wegen geschehen.

Aus dem Stand der Technik ist eine Reihe von Verfahren bekannt, die sich beispielsweise zur Herstellung der erfindungsgemäßen lonomerteilchen nutzen lassen.

Stöber und Fink (Journal of Colloid and Interface Science 26, 62-69 (1968)) beschrieben die Herstellung von agglomeratfreien, monodispersen Silica-Partikeln ausgehend von Tetraalkoxysilanen und Wasser in Alkohol unter ammoniakalischer Katalyse (allg. als Stöber-Prozeß bekannt). Die Partikelgröße kann über verschiedene Parameter wie Wasser-, Ammoniak-Konzentration, Wahl des Alkoxysilans und Temperatur im Bereich von 50 bis 2000 nm eingestellt werden.

Die EP 216 278 beschreibt ein Verfahren auf Basis des Stöber-Prozesses zur Herstellung von monodispersen, unporösen Silica-Partikeln der Größe 50 bis 10.000 nm, deren Monodispersität auf unter 5% optimiert wurde.

Auf der Grundlage des Stöber-Prozesses beschreibt die DE PS 3247800 die Herstellung von kugelförmigen, amorphen Mischpartikeln auf Basis von Silica und 0,01 bis 20 mol% eines Metalloxides der I.-IV. Gruppe des Periodensystems.

Die Patentschrift DE 38 34 774 liefert eine Übersicht über den Stand der Technik des Emulsionsverfahrens für Wasser-in-Öl-Emulsionen (W/O). Minehan und Messing (Colloids and Surfaces 63, 181 bis 187 (1992)) beschreiben die Herstellung von Silica-Partikeln über Öl-In-Wasser-Emulsion (O/W). Zur Erzeugung von Nanopartikeln (<100 nm) sind Mikroemulsionsverfahren bekannt, wie z.B. die Herstellung von SiO₂-Partikeln nach K. Osseo-Asare, F.J. Arrigada, Colloids and Surfaces 50, 321-339 (1990) in nichtionischen Mehrkomponentensystemen. Partikel des Größenbereiches 50-70 nm werden mit Standardabweichungen von weniger als 8,5% erhalten.

Verschiedene thermolytische Aerosolverfahren (engl.: solution aerosol thermolysis (SAT)) wie z.B. die Sprühtrockung sind zur Herstellung von Pulvern bekannt (G.V. Jayanthi, S.C. Zhang, G.L. Messing, Aerosol Science and Technology, 19, 478-490 (1993)). Die SAT stellt eine Klasse von Herstellungsverfahren dar, bei denen eine Precursor-Flüssigkeit mit Hilfe von Düsen in einen vorgeheizten Ofen gesprüht wird. Alternativ kann auch ein Schwingblendengenerator verwendet werden (R.N. Berglund et al., Environ. Sci. Technol. 1973, 7 (2), 147-153). Die entstehenden Tröpfchen liegen je nach Durchmesser der Düse bzw. der Generatorfrequenz im Bereich von weniger als 1 µm bis hin zu mehr als 50 µm. Das Lösungsmittel verdampft bei den hohen Temperaturen im Ofen, so daß trockene Pulver entstehen.

Die genannten Verfahren lassen sich prinzipiell zur Herstellung der erfindungsgemäßen sphärischen oder annähernd sphärischen Ionomerpartikel nutzen, wobei anstelle der dortigen hydrolysierbaren Verbindungen die organischen und ggf. sonstigen Verbindungen in die Flüssigkeit eingebracht werden müssen, die in der vorliegenden Erfindung wesentlich sind.

Erfindungsgemäß gelingt es nun beispielsweise mit Hilfe eines auf dem Stöber-Prozeß basierenden Verfahrens, auf verschiedene inerte Partikelkerne (z.B. SiO₂-, SnO₂-Kerne) eine Siliciumionen enthaltende Schale aufzubringen, die zusätzliche Elemente der Gruppe a) und b) enthält. Als Kerne können beliebig hergestellte monodisperse, kugelförmige Keime eingesetzt werden. U.a. eignen sich kommerziell erhältliche, agglomeratfreie, monodisperse, kugelförmige SiO₂-(z.B. Ludox, Fa. DuPont) oder SnO₂-Partikel. In Anlehnung an den oben genannten Stöber-Prozeß können auch monodisperse kugelförmige SiO₂-Kerne im Größenbereich von 50 bis 2000 nm hergestellt werden, die dann mit einer "Schale" versehen werden.

Zum Aufbringen der Schale können als Ausgangsverbindungen siliciumorganische Verbindungen wie beispielsweise Alkoxysilane in Kombination mit Verbindungen der Gruppe a) und b) (die beiden letzteren in organischer oder anorganischer Form) eingesetzt werden. Die organischen Verbindungen oder ein niedermolekulares Kondensationsprodukt davon werden z.B. zu 1-40 Gew.-% einem Lösungsmittel, vorzugsweise einem Alkohol, hinzugegeben. Diese Lösung wird in der Weise zur Mutter-Dispersion der Kerne hinzutitriert, daß im Verlauf des Wachstumsprozesses der Partikel eine Übersättigungskonzentration, die zur Neubildung von Teilchen führen würde, nicht erreicht wird. Da nach diesem Verfahren die organischen Verbindungen hydrolysiert werden sollen, wird Wasser in einer Konzentration zugegeben, die an die Konzentration der Edukte angepaßt ist. Da die Hydrolyse-/Kondensationsreaktionen unter neutralen Bedingungen sehr langsam ablaufen, ist ein saures bzw. alkalisches Milieu bevorzugt. Im Rahmen dieser Erfindung wurde gefunden, daß ein pH 8-9 zum gleichmäßigen Wachstum der Partikel von Vorteil ist und nahezu ideal kugelförmige, monodisperse lonomerpartikel resultieren. Diese zeichnen sich durch eine überraschend schnelle lonomerreaktion aus.

Eine in situ-Oberflächenmodifizierung gelingt u.a. durch Zugabe eines Silans, z.B. von Aminopropyltriethoxysilan oder Methacryloxypropyltrimethylsilan, in Form einer 1-100 gew.-%igen Lösung zur Dispersion. Als Lösungsmittel wird vorzugsweise das gleiche Lösungsmittel der Mutter-Dispersion verwendet, beispielsweise Ethanol. Ebenfalls möglich ist eine nachträgliche Obeflächenmodifizierung der getrockneten Partikel. Dazu wird das Partikelpulver zu ca. 10 Gew.-% in einem organischen Lösungsmittel, z.B. Toluol, suspendiert, mit der zur monomolekularen Belegung notwendigen Menge an Silan versetzt, ggf. ein Katalysator hinzugefügt und ggf. unter Rückfluß gekocht.

Der Stöber-Prozeß kann selbstverständlich auch zur Herstellung homogener oder anderer Partikel ohne Kern eingesetzt werden. Die Bindungen müssen dann so gewählt werden, daß die Teilchenbildung induziert wird.

Weiterhin wurde gefunden, daß sich auch Emulsionsverfahren sehr gut zur Herstellung der oben beschriebenen lonomerpartikel eignen. Verwendbar sind das O/W- sowie das W/O-Verfahren. Vorzugsweise wird das W/O-Verfahren eingesetzt (s. z.B.
EP 0363927). Der Anteil der wäßrigen Phase liegt bevorzugt bei ca. 15 bis 45 Vol.-%, der des Emulgators bevorzugt bei ca. 1 bis 20 Gew.-%. In den Wassertröpfchen findet im Verlauf des Emulsionsverfahrens eine Ausfällung oder Gelbildung statt, die vorzugsweise durch eine basische pH-Wert-Verschiebung eingeleitet wird. Als Ausgangsverbindungen eignen sich Salze und organische Komplexe der bereits oben beschriebenen Elemente, bevorzugt Nitrate, Alkoholate und Acetate, sowie bereits daraus hergestellte Dispersionen ohne Einschränkung. Die erhaltenen lonomerpartikel weisen überraschenderweise eine enge Größenverteilung auf, die deutlich unter einer Zehnerpotenz liegen kann.

Die voranstehend beschriebene Flüssigkeit kann statt dessen aber auch einer Aerosolbehandlung, insbesondere einer Sprühtrocknung, unterworfen werden. Beispielsweise können sehr fein disperse SiO₂-Partikel oder Siliciumalkoxide mit Alkoholaten oder Carboxylaten der Kationen der Gruppen a) und b) in wäßriger Lösung mit pH < 7 vermischt werden. Mit Hilfe geeigneter Düsen werden Tröpfchen versprüht, die kugelförmige Gestalt aufweisen. Diese können ggf. getrocknet werden, beispielsweise bei etwa 250°C, bis die flüchtigen organischen Bestandteile entfernt sind.

Allen Verfahren ist gemeinsam, daß die erhaltenen Partikel nach Entfernen des Lösungsmittels oder nach Abtrennen aus dem Lösungsmittel auf Wunsch einer Pyrolyse unterworfen werden können, sofern noch organische Bestandteile vorhanden sind (beispielsweise bei 400° bis 600°C). Hierdurch entstehen organikfreie silikatische lonomerpartikel.

Je nach den eingesetzten Ausgangsverbindungen werden bei dem vorgenannten Verfahren Ionomerpartikel unterschiedlicher Struktur gebildet. Die Partikel können einen durchgehend homogenen Bereich aufweisen oder aber silikathaltige Bereiche neben Calciumalumosilikaten, Strontiumalumosilikaten oder dergleichen bzw. ausschließlich Silicium als Kationen enthaltende Bereiche neben Calcium und/oder Strontium, Aluminium und Silicium oder dergleichen enthaltende Bereiche aufweisen. Die Ionomerpartikel können ausschließlich aus diesen Strukturen bestehen oder aber einen diskreten inneren Bereich aufweisen, der eine andere Zusammensetzung besitzt, beispielsweise Siliciumdioxid, Zinndioxid, eine Mischung aus beidem, Aluminiumsilikat oder dergleichen. In einer spezifischen Ausgestaltung bestehen die sphärischen lonomerpartikel aus einem inneren Bereich und mehreren äußeren, vorzugsweise schalenförmigen Bereichen. Diese können beispielsweise dadurch hergestellt werden, daß Siliciumdioxid-Partikel geeigneter Größe mit einem ersten Gel oder Sol umhüllt, getrocknet und ggf. pyrolysiert werden, worauf die entstandenen Partikel mit einem zweiten Gel oder Sol anderer Zusammensetzung umhüllt, wiederum getrocknet und ggf. pyrolysiert werden. Mindestens das zweite Gel oder Sol muß dabei eine Zusammensetzung wie voranstehend beschrieben aufweisen. Obwohl dies im allgemeinen nicht notwendig sein wird, lassen sich die erfindungsgemäßen sphärischen lonomerpartikel auch in üblicher Weise silanisieren oder anderweitig oberflächenmodifizieren.

Werden die erfindungsgemäßen lonomere in Matrixsysteme, bevorzugt in säurehaltige Matrixsysteme eingearbeitet, resultieren nach der oben beschriebenen zweistufigen Härtung neuartige Werkstoffe, z.B. Komposite, Zemente, Kompomere. Deren Eigenschaften lassen sich, wie beschrieben, durch Einsatz entsprechender Ausgangssubstanzen gezielt einstellen, z.B. durch Zusatz röntgenopaker Bestandteile oder durch Reaktionsbedingungen (z.B. Konzentration, Temperatur, pH), mit denen sich der Durchmesser der Teilchen variieren läßt. Diese Werkstoffe sind insbesondere im zahnärztlichen Bereich (z.B. als Füllungsmaterial) und im Medizinsektor (z.B. als Knochenzement) einsetzbar. Weiterhin sind Materialien mit unterschiedlicher Transparenz, Farbe, unterschiedlichem Brechungsindex einstellbar.

Überraschend hat sich herausgestellt, daß die sphärische Gestalt und Größe der erfindungsgemäßen lonomerpartikel zu einer wesentlich besseren Abriebfestigkeit und Mechanik von Zahnfüllungen führt, wenn sie zusammen mit Alkensäuren zu einem Zement verarbeitet werden. Weiterhin wurde überraschenderweise festgestellt, daß die Geschwindigkeit der lonomerreaktion und der Zementbildung stark erhöht ist und die Stärke der Reaktion zugenommen hatte. Dies läßt sich anhand von IR-Spektroskopie nachweisen.

Als Matrixsysteme, mit denen die erfindungsgemäßen Ionomerpartikel zu Zementen verarbeitet werden können, eignen sich die bereits erwähnten Alkensäuren, z.B. Polyalkensäuren (Mono-, Di- oder Tricarbonsäuren) wie Polyacrylsäure, Polyitaconsäure, Polymaleinsäure oder Copolymere der entsprechenden Säuren, denen ggf. Hydroxycarbonsäuren wie z.B. Citronensäure oder Weinsäure zugegeben werden kann. Diese können in wäßriger Phase oder gefriergetrocknet bereitgestellt werden; in letzterem Zustand muß beim Anmischen mit den lonomeren natürlich Wasser zugesetzt werden. Aber auch andere, bevorzugt saure Matrixsysteme sind möglich, z.B. Polyphosphonsäuren wie Poly-(vinylphosphonsäure), Systeme die zusätzlich lichthärtbare Bestandteile enthalten oder Matrices, die mit lonomerpartikeln die oben beschriebenen Kompomere bilden können.

### Beispiel 1 (SiO₂/CaO/Al₂O₃-Partikel, Molverhältnis: Si/Ca/Al-1:1:1)

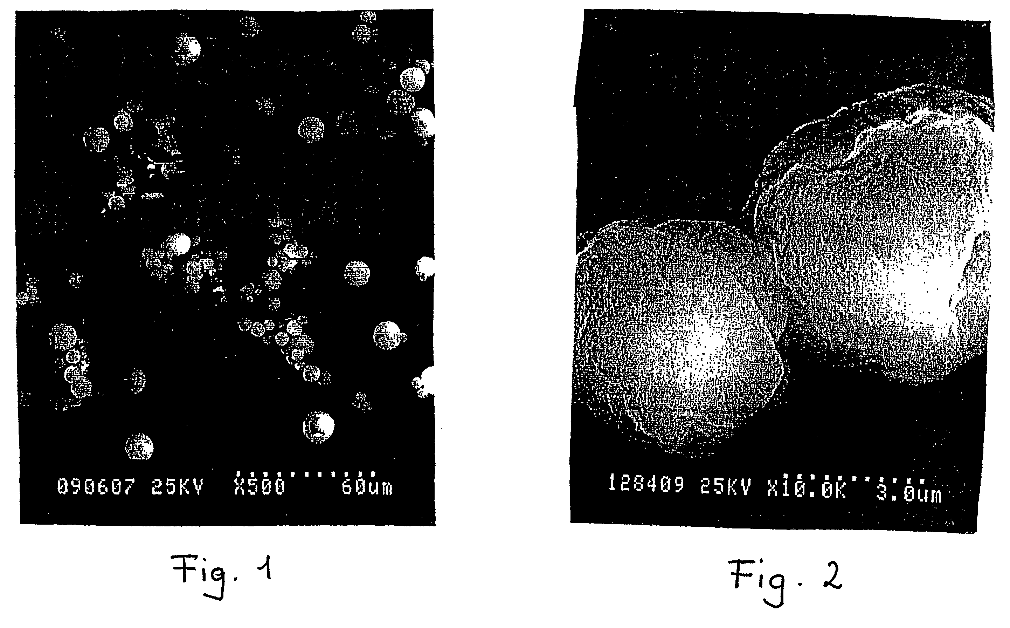

7,6 g Aluminium-sec.-butylat wurden bei RT unter Rühren mit 40 ml Wasser und Eisessig versetzt. Dazu wurde unter Rühren eine Lösung aus 4,7 g Calciumacetat in 20 ml Wasser und 1 ml konz. Essigsäure gegeben. Zu dieser resultierenden Mischung wurde eine Dispersion getropft, die durch eine Verdünnung von 5 g kommerziell erhältlichen SiO₂-Partikeln (Ludox AS 40) mit 45 g Wasser und Zutropfen von 1 ml konz. Essigsäure erhalten wurde. Nach der Sprühtrocknung bei etwa 250°C wurde ein weißes Pulver erhalten, das gemäß REM-Untersuchung aus kugelförmigen Partikeln besteht. RFA-Untersuchungen bestätigten ein Si/Al/Ca-Verhältnis, das dem Eduktverhältnis gleicht. Eine Temperaturbehandlung von 3h bei 500°C folgte. Figur 1 zeigt eine REM-Aufnahme der entstandenen Teilchen.

Zum Nachweis der Zementbildung und somit der Funktionsfähigkeit der synthetisierten Partikel wurden diese in ein Carbonsäure-haltiges Harz eingearbeitet und einer Wasserlagerung unterzogen. Es trat eine Härtung der Mischung im Rahmen einer lonomerreaktion ein, die mit Hilfe der FTIR-Spektroskopie der sich bildenden COO-AIund COO-Ca-Bindungen anhand von Schwingungen bei ca. 1560 bis 1450 bis 1410 cm⁻¹ nachgewiesen wurden.

### Beispiel 2 (SiO₂/SnO₂/SrO/Al₂O₃-Partikel; Molverhältnis: Si/Sn/Sr/Al=1:1:1:2)

14,2 g Aluminium-sec.-butylat wurden bei RT unter Rühren mit 80 ml Wasser und Eisessig versetzt. Unter Rühren wurde eine Lösung aus 5,93 g Strontiumacetat in 50 ml Wasser und 1 ml konz. Essigsäure dazugegeben. Dazu wurde langsam eine Mischung aus 4,33 g einer kommerziell erhältlichen 40 Gew.-% wäßrigen SiO₂-Dispersion, 28,9 g einer kommerziell erhältlichen 15 Gew.-% wäßrigen SnO₂-Dispersion, 150 ml Wasser und 1 ml konz. Essigsäure getropft. Nach der Sprühtrocknung bei etwa 250°C wurde ein weißes Pulver erhalten. RFA-Untersuchungen bestätigten ein Si/Sn/Sr/Al-Verhältnis, das dem Eduktverhältnis gleicht. Eine Temperaturbehandlung von 3h bei 500°C folgte. Figur 2 zeigt eine REM-Aufnahme der entstandenen Teilchen.

Zum Nachweis der Zementbildung und somit der Funktionsfähigkeit der sythetisierten Partikel wurden diese in ein Carbonsäure-haltiges Harz eingearbeitet und einer Wasserlagerung unterzogen. Es trat eine Härtung der Mischung im Rahmen einer lonomerreaktion ein, die mit Hilfe der FTIR-Spektroskopie der sich bildenden COO-Alund COO-Sr-Bindungen anhand von Schwingungen bei ca. 1555 bzw. 1455 bis 1400 cm⁻¹ nachgewiesen wurde.

### Beispiel 3 (SiO₂-Keime und Beschichtung mit Si/Ca/A1-haltigen Precursoren)

### 3.1. Herstellung monodisperser SiO₂-Keime

45 ml 25% Ammoniak werden 900 ml Ethanol in einen 1I-Erlenmeyerkolben gegeben und gerührt. Zu dieser auf 25°C temperierten Lösung werden 45 g (0,2 mol) von gleichermaßen temperiertem Tetraethoxysilan gegeben. Es wird eine Dispersion mit Partikeln einer mittleren Größe von 60 nm bei einer Standardabweichung von < 10% erhalten (dynamische Lichtstreuung, Transmissionelektronenmikroskopie).

### 3.2. Beschichtung mit Si/Ca/Al-haltigen Precursoren

1 g der unter 3.1 beschriebenen SiO₂-Keime werden in 70 ml wasserfreiem Isopropanol dispergiert. Der pH-Wert wird unter Verwendung von 0,1 n HCI-Lösung auf einen Wert von 9 eingestellt. Parallel dazu stellt man eine Lösung B her, indem 25 mg Ca unter Inertgasatmosphäre mit 130 ml Isopropanol versetzt, 1 h bei Raumtemperatur gerührt und anschließend zum Sieden erhitzt werden. 140 mg Aluminium-sec.-butylat und 1,0 g Tetraethoxysilan werden hinzugetropft und das ganze unter Rückfluß erhitzt. Nach Abkühlung auf RT wird Lösung B zur Partikeldispersion gegeben und danach noch 10 h weitergerührt. Der anschließende Waschvorgang schließt eine zweimalige Redispergierung in Isopropanol sowie die Isolierung mittels Zentrifugation ein. RFA-Untersuchungen bestätigen ein Si/Ca/Al-Verhältnis, das dem Eduktverhältnis gleicht. Die mittlere Größe der Partikel liegt bei 75 nm bei einer Standardabweichung von < 10% (DLS).

Zum Nachweis der Zementbildung und somit der Funktionsfähigkeit der synthetisierten Partikel werden diese in ein Carbonsäure-haltiges Harz eingearbeitet und einer Wasserlagerung unterzogen. Es tritt eine Härtung der Mischung im Rahmen einer lonomerreaktion ein, die mit Hilfe der FTIR-Spektroskopie der sich bildenden COO-AIund COO-Ca-Bindungen anhand von Schwingungen bei ca. 1560 bzw. 1450 bis 1410 cm⁻¹ nachgewiesen wird.

### Beispiel 4 (SiO₂-Keime und Beschichtung mit Si/Sr/Al-haltigen Precursoren

### 4.1. Herstellung monodisperser Keime

18 ml 25% Ammoniak und 12 ml destilliertes Wasser werden zu 360 ml Ethanol in einen 500 ml-Erlenmeyerkolben gegeben und gerührt. Zu dieser auf 25°C temperierten Lösung werden 18 g von gleichermaßen temperiertem Tetraethoxysilan gegeben. Es wird eine Dispersion mit Partikeln einer mittleren Größe von 185 nm bei einer Standardabweichung von <10% erhalten (dynamische Lichtstreuung, Transmissionselektronenmikroskopie).

### 4.2. Beschichtung mit Si/Sr/Al-haltigen Precursoren

1g der unter 4.1. beschriebenen SiO₂-Keime werden in 70 ml wasserfreiem Isopropanol dispergiert. Der pH-Wert wird unter Verwendung von 0,1 n HCl-Lösung auf einen Wert von 9 eingestellt. Parallel dazu stellt man eine Lösung B her, indem 50 mg Sr unter Inertgasatmosphäre mit 130 ml Isoprpanol versetzt, 1 h bei Raumtermperatur gerührt und anschließend zum Sieden erhitzt werden. 140 mg Aluminium-sec.-butylat und 1,0 g Tetraethoxysilan werden hinzugetropft und das ganze unter Rückfluß erhitzt. Nach Abkühlung auf RT wird Lösung B zur Partikeldispersion gegeben und danach noch 10 h weitergerührt. Der anschließende Waschvorgang schließt eine zweimalige Redispergierung in Isopropanol sowie die Isolierung mittels Zentrifugation ein. RFA-Untersuchungen bestätigen ein Si/Sr/Al-Verhältnis, das dem Eduktverhältnis gleicht. Die mittlere Größe der Partikel liegt bei 190 nm bei einer Stardardabweichung von <10% (DLS).

Zum Nachweis der Zementbildung und somit der Funktionsfähigkeit der synthetisierten Partikel werden diese in ein Carbonsäure-haltiges Harz eingearbeitet und einer Wasserlagerung unterzogen. Es tritt eine Härtung der Mischung im Rahmen einer lonomerreaktion ein, die mit Hilfe der FTIR-Spektroskopie der sich bildenden COO-AIund COO-Sr-Bindungen anhand der Schwingungen bei ca. 1555 bzw. 1455 bis 1400 cm⁻¹ nachgewiesen wird.

### Beispiel 5 (SiO₂-Keime und Beschichtung mit Si/Sr/Al-haltigen Precursoren)

### 5.1. Herstellung monodisperser SiO₂-Keime: Siehe Beispiel 3.1.

### 5.2. Beschichtung mit Si/Sr/Al-haltigen Precursoren

1 g der in Beispiel 3.1. beschriebenen SiO₂-Keime werden in 70 ml wasserfreiem Isopropanol dispergiert. Der pH-Wert wird unter Verwendung von 0,1 n HCI-Lösung auf einen Wert von 9 eingestellt. Parallel dazu stellt man eine Lösung B her, indem 50 mg Sr unter Inertgasatmosphäre mit 130 ml Isopropanol vesetzt, 1 h bei Raumtemperatur gerührt und anschließend zum Sieden erhitzt werden. 140 mg Aluminium-sec.-butylat und 1,0 g Tetraethoxysilan werden hinzugetropft und das ganze unter Rückfluß erhitzt. Nach Abkühlung auf RT wird Lösung B zur Partikeldispersion gegeben und danach noch 10 h weitergerührt. Der anschließende Waschvorgang schließt eine zweimalige Redispergierung in Isopropanol sowie die Isolierung mittels Zentrifugation ein. RFA-Untersuchungen bestätigen ein Si/Sr/Al-Verhältnis, das dem Eduktverhältnis gleicht. Die mittlere Größe der Partikel liegt bei 75 nm bei einer Standardabweichung von < 10% (DLS).

Zum Nachweis der Zementbildung und somit der Funktionsfähigkeit der synthetisierten Partikel werden diese in ein Carbonsäure-haltiges Harz eingearbeitet und einer Wasserlagerung unterzogen. Es tritt eine Härtung der Mischung im Rahmen einer lonomerreaktion ein, die mit Hilfe der FTIR-Spektroskopie der sich bildenden COO-AIund COO-Sr-Bindungen anhand von Schwingungen bei ca. 1555 bzw. 1455 bis 1400 cm⁻¹ nachgewiesen wird.

### Beispiel 6 (Beschichtung von SnO₂-Keimen mit Si/Sr/Al-haltigen Precursoren)

1 g kommerziell erhältlicher SnO₂-Keime (⌀ 10 nm) werden in 50 ml Wasser dispergiert und mit 250 ml Isopropanol verdünnt. Der pH-Wert wird unter Verwendung von 0,1n HCI-Lösung auf 9 eingestellt. Parallel dazu stellt man eine Lösung B her, indem 50 mg Sr unter Intergasatmosphäre mit 130 ml Isopropanol versetzt, 1 h bei Raumtemperatur gerührt und anschließend zum Sieden erhitzt werden. 140 mg Aluminium-sec.-butylat und 1,0 g Tetraethoxysilan werden hinzugetropft und das ganze unter Rückfluß erhitzt. Nach Abkühlung auf RT wird Lösung B zur Partikeldispersion gegeben und danach noch 10 h weitergerührt. Der anschließende Waschvorgang schließt eine zweimalige Redispergierung in Isopropanol sowie die Isolierung mittels Zentrifugation ein. RFA-Untersuchungen bestätigen ein Si/Sn/Sr/Al-Verhältnis, das dem Eduktverhältnis gleicht. Die mittlere Größe der Partikel liegt bei 20 nm bei einer Standardabweichung von < 10% (DLS).

Zum Nachweis der Zementbildung und somit der Funktionsfähigkeit der synthetisierten Partikel werden diese in ein Carbonsäure-haltiges Harz eingearbeitet und einer Wasserlagerung unterzogen. Es tritt eine Härtung der Mischung im Rahmen einer Ionomerreaktion ein, die mit Hilfe der FTIR-Spektroskopie der sich bildenden COO-AIund COO-Sr-Bindungen anhand von Schwingungen bei ca. 1555 bzw. 1455 bis 1400 cm⁻¹ nachgewiesen wird.

Es wurde gefunden, daß die Röntgenabsorption der Ionomerpartikel in der Reihenfolge der in Beispiel 3 bis Beispiel 6 angegebenen Zusammensetzung zunimmt. Dies steht in Übereinstimmung mit dem Einbau der zunehmend schwereren Elemente in der Reihe Ca (Bsp. 3), Sr (Bsp. 4 und 5) und zuletzt Sn (Bsp. 6).

### Beispiel 7 (Oberflächenmodifizierung)

1 g der in Beispiel 5 erhaltenen lonomerpartikel werden in 100 g Toluol dispergiert, 2 g Methacryloxypropyltriethoxysilan hinzugegeben und 5 h zum Sieden erhitzt. Nach Abkühlen auf RT werden die Partikel mittels Zentrifugation isoliert und zweimal mit Toluol über Redispersion/Zentrifugation-Zyklen gewaschen. Die Trockunun gerfolgte über 7 h bei 100°C im Ölpumpenvakuum. Die Modifizierung wird mittels der diffusen Reflexion-Infrarot-Fourier-Transformations-Spektroskopie (DRIFTS) anhand einer für C=O- und C=C-Doppelbindungen spezifischen Schwingung bei 1720 und 1636 cm⁻¹ nachgewiesen.

## Patentansprüche

1. Sphärische oder annähernd sphärische Ionomerpartikel mit einem inneren Bereich und einem äußeren, Siliciumionen enthaltenden Bereich, dessen Kationen (a) mindestens ein Element, das in silikatischen Verbindungen Gitterplätze des Siliciums unter Entstehen eines negativen Ladungsüberschusses einnehmen kann, und (b) mindestens ein Element umfassen, das aus den Elementen der ersten und zweiten Hauptgruppe sowie solchen, die in zweiwertiger Form auftreten können, ausgewählt ist und den negativen Ladungsüberschuß kompensieren kann.

2. Sphärische Ionomerpartikel nach Anspruch 1, worin der äußere Bereich ein oxidischer Bereich ist.

3. Sphärische lonomerpartikel nach Anspruch 1 oder 2, worin der innere und der äußere Bereich identisch sind.

4. Sphärische lonomerpartikel nach einem der Ansprüche 1 bis 3 mit einer homogenen Verteilung der Bestandteile.

5. Sphärische Ionomerpartikel nach einem der Ansprüche 1 bis 3 mit clusterartigen Bereichen inerter Bestandteile.

6. Sphärische lonomerpartikel nach Anspruch 1 oder 2 worin sich der innere Bereich chemisch vom äußeren Bereich unterscheidet.

7. Sphärische lonomerpartikel nach Anspruch 6, worin der innere Bereich aus Siliciumdioxid und/oder Zinndioxid besteht.

8. Sphärische lonomerpartikel nach einem der voranstehenden Ansprüche, in denen die Kationen der Gruppe (a) Aluminiumkationen sind und die Kationen der Gruppe (b) Calciumionen und/oder Strontiumionen sind.

9. Sphärische lonomerpartikel nach einem der voranstehenden Ansprüche, worin der äußere Bereich durch Silanisierung oberflächenmodifiziert ist.

10. Verfahren zum Herstellen sphärischer oder annähernd sphärischer lonomerpartikel nach einem der Ansprüche 1 bis 8, umfassend die folgenden Schritte:
(i) Bilden einer Dispersion, einer Suspension, einer Lösung, einer Emulsion, eines Gels oder eines Sols unter Einsatz von
(1) mindestens einer organischen Komponente, die ausgewählt ist unter siliciumorganischen Verbindungen und organischen Verbindungen der Kationen der in Anspruch 1 unter (a) und (b) genannten Elemente, sowie
(2) sofern unter (1) keine siliciumorganische Verbindung eingesetzt wird, Siliciumdioxid und/oder mindestens einem anorganischen Salz des Siliciums, und
sofern unter (1) keine organische Verbindung eines unter (a) genannten Elementes eingesetzt wird, mindestens einem Oxid und/oder einem anorganisches Salz dieses Elementes, und sofern unter (1) keine organische Verbindung eines unter (b) genannten Elementes eingesetzt wird, mindestens einem Oxid und/oder einem anorganisches Salz dieses Elementes,
in einem geeigneten flüssigen Medium,
(ii) Bewirken einer zumindest teilweisen Hydrolyse und Kondensation der unter (1) genannten Komponente(n).
(iii) Erzeugen und ggf. Abtrennen sphärischer oder annähernd sphärischer Partikel in bzw. aus dem flüssigen Medium, und
(iv) Trocknen der sphärischen oder annähernd sphärischen Partikel.

11. Verfahren nach Anspruch 10, weiterhin umfassend den folgenden Schritt:
Erhitzen der getrockneten Partikel auf mindestens eine solche Temperatur, bei der ggf. noch vorhandene organische Bestandteile der Partikel entfernt werden.

12. Verfahren nach Anspruch 10 oder 11, worin die Partikel durch ein Aerosolverfahren, insbesondere durch Sprühtrocknung, erzeugt werden.

13. Verfahren nach Anspruch 10, worin die Dispersion, Suspension, das Gel oder Sol zusätzlich unter Einsatz von oxidischen Partikeln erfolgt, deren Durchmesser unter dem der herzustellenden lonomerpartikeln liegt, bevorzugt um einen Bereich von 1-10%.

14. Verfahren nach Anspruch 10, zusätzlich umfassend die Schritte:
(o) Erzeugen einer Partikeldispersion durch vorsichtige, ggf. säure- oder basenkatalysierte Hydrolyse eines Metallalkoxids in alkoholischer Lösung, Abtrennen der erhaltenen Partikel aus der Suspension, ggf. Trocknen der Partikel, ggf. Erhitzen der Partikel, um noch vorhandenes organisches Material auszutreiben, und Einsetzen der Partikel zur Gewinnung der Dispersion, Suspension, des Gels oder Sols gemäß (1) in Anspruch 10.

15. Verfahren nach einem der Ansprüche 10 bis 12, worin die Partikel durch Erzeugen einer Emulsion, einer Dispersion oder einer Suspension gebildet werden, worauf sie aus dem umgebenden Lösungsmittel abgetrennt werden oder das umgebende Lösungsmittel entfernt wird.

16. Verfahren nach einem der Ansprüche 10 bis 15, worin die organische(n) Komponente(n) ausgewählt ist/sind unter Alkoxysilanen, Aluminiumalkoholat und Calciumacylat.

17. Verfahren nach Anspruch 16, worin die organischen Komponenten unter Aluminiumbutylat und Calciumacetat ausgewählt sind und weiterhin Siliciumdioxid eingesetzt wird.

18. Verwendung sphärischer oder annähernd sphärischer lonomerpartikel nach einem der Ansprüche 1 bis 9 in Dentalzementen, Knochenzementen oder Klebern.

19. Verwendung sphärischer oder annähernd sphärischer lonomerpartikel nach einem der Ansprüche 1 bis 9 oder 18 zusammen mit Polysäuren, insbesondere Polyalkensäuren als Zement.

## Claims

1. Spherical or approximately spherical ionomer particles with an interior region and an exterior region which contains silicon ions, the cations of which include (a) at least one element which in siliceous compounds can occupy lattice positions of silicon in the event of a negative charge excess, and (b) at least one element which is chosen from the elements of the first and second principal group and also those which can occur in bivalent form and can compensate for the negative charge excess.

2. Spherical ionomer particles according to claim 1, in which the exterior region is an oxidic region.

3. Spherical ionomer particles according to claim 1 or 2, in which the interior and the exterior region are identical.

4. Spherical ionomer particles according to one of the claims 1 to 3, with a homogenous distribution of the components.

5. Spherical ionomer particles according to one of the claims 1 to 3, with cluster-type regions of inert components.

6. Spherical ionomer particles according to claim 1 or 2, in which the interior region differs chemically from the exterior region.

7. Spherical ionomer particles according to claim 6, in which the interior region comprises silicon dioxide and/or tin dioxide.

8. Spherical ionomer particles according to one of the preceding claims, in which the cations of the group (a) are aluminium cations and the cations of the group (b) are calcium ions and/or strontium ions.

9. Spherical ionomer particles according to one of the preceding claims, in which the exterior region is surface-modified by means of silanisation.

10. Method for producing spherical or approximately spherical ionomer particles according to one of the claims 1 to 8, comprising the following steps:
(i) formation of a dispersion, a suspension, a solution, an emulsion, a gel or a sol using
(1) at least one organic component which is chosen from organosilicon compounds and organic compounds of the cations of the elements mentioned in claim 1 under (a) and (b), and
(2) provided that under (1) no organosilicon compound is used, silicon dioxide and/or at least one inorganic salt of silicon, and
provided that under (1) no organic compound of an element mentioned under (a) is used, at least one oxide and/or one inorganic salt of this element, and
provided that under (1) no organic compound of an element mentioned under (b) is used, at least one oxide and/or an inorganic salt of this element,
in a suitable liquid medium.
(ii) effecting an at least partial hydrolysis and condensation of the component(s) mentioned under (1).
(iii) production and, if necessary, separation of spherical or approximately spherical particles in or from the liquid medium, and
(iv) drying of the spherical or approximately spherical particles.

11. Method according to claim 10, furthermore comprising the following step:
heating of the dried particles to at least such a temperature at which the possibly still present organic components of the particles are removed.

12. Method according to claim 10 or 11, in which the particles are produced by means of an aerosol method, in particular by means of spray drying.

13. Method according to claim 10, in which the dispersion, suspension, the gel or sol is effected additionally with the use of oxidic particles, the diameter of which is below that of the ionomer particles to be produced, preferably by a range of 1 to 10%.

14. Method according to claim 10, additionally comprising the steps:
(o) production of a particle dispersion by means of careful, if necessary acid- or base-catalysed hydrolysis of a metal alkoxide in alcoholic solution, separation of the obtained particles from the suspension, if necessary drying of the particles, if necessary heating of the particles, in order to expel still present organic material, and use of the particles for obtaining the dispersion, suspension, the gel or sol according to (1) in claim 10.

15. Method according to one of the claims 10 to 12, in which the particles are formed by producing an emulsion, a dispersion or a suspension, whereupon they are separated from the surrounding solvent or the surrounding solvent is removed.

16. Method according to one of the claims 10 to 15, in which the organic component(s) is/are chosen from alkoxysilanes, aluminium alcoholate and calcium acylate.

17. Method according to claim 16, in which the organic components are chosen from aluminium butylate and calcium acetate and furthermore silicon dioxide is used.

18. Use of spherical or approximately spherical ionomer particles according to one of the claims 1 to 9 in dental cements, bone cements or adhesives.

19. Use of spherical or approximately spherical ionomer particles according to one of the claims 1 to 9 or 18 together with polyacids, in particular polyalkene acids as cement.

## Revendications

1. Particules ionomères sphériques ou presque sphériques ayant une zone interne et une zone externe, contenant des ions silicium, dont les cations comprennent (a) au moins un élément qui peut prendre dans les composés silicatés des places réticulaires du silicium avec apparition d'un excès de charge négative et (b) au moins un élément qui est choisi parmi ceux des premiers et seconds groupe de la classification périodique des éléments ainsi que ceux qui peuvent apparaître sous une forme bivalente, et qui peuvent compenser l'excès de charge négative.

2. Particules ionomères sphériques selon la revendication 1, dont la zone externe est dans le domaine des oxydes.

3. Particules ionomères sphériques selon la revendication 1 ou 2, dans laquelle la zone interne et la zone externe sont identiques.

4. Particules ionomères sphériques selon l'une des revendications 1 à 3 ayant une répartition homogène des constituants.

5. Particules ionomères sphériques selon l'une des revendications 1 à 3 avec domaines en amas de constituants linéaires.

6. Particules ionomères sphériques selon l'une des revendications 1 ou 2, où la zone interne se différencie chimiquement de la zone externe.

7. Particules ionomères sphériques selon la revendication 6, dans laquelle la zone externe se compose de dioxyde de silicium et/ou de dioxyde d'étain.

8. Particules ionomères sphériques selon l'une des revendications précédentes, dans lesquelles les cations du groupe (a) sont des cations aluminium et les cations du groupe (b) sont des ions calcium et/ou des ions strontium.

9. Particules ionomères sphériques selon l'une des revendications précédentes, dans lesquelles la zone externe a ses surfaces modifiées par silanisation.

10. Procédé de préparation de particules ionomères sphériques ou presque sphériques selon l'une des revendications 1 à 8, comprenant les étapes suivantes :
(i) formation d'une dispersion, d'une suspension, d'une solution, d'une émulsion, d'un gel ou d'un sol avec utilisation de
(1) au moins un composant organique qui est choisi parmi les composés d'organosilicium et les composés organiques des cations des éléments mentionnés dans la revendication 1 en (a) et (b) ainsi que
(2) dans la mesure où l'on n'utilise pas de composé d'organosilicium en (1), du dioxyde de silicium et/ou au moins un sel inorganique de silicium, et
dans la mesure où l'on n'utilise pas de composé organique d'un élément mentionné en (a), au moins un oxyde et/ou un sel inorganique de cet élément, et dans la mesure où l'on n'utilise pas de composé organique d'un élément mentionné sous (b), au moins un oxyde et/ou un sel inorganique de cet élément,
dans un milieu liquide approprié ;
(ii) déclenchement d'une hydrolyse au moins partielle et d'une condensation du ou des composants mentionnés en (1),
(iii) production et le cas échéant séparation des particules sphériques ou presque sphériques dans ou selon les cas à partir du milieu liquide, et
(iv) séchage des particules sphériques ou presque sphériques.

11. Procédé selon la revendication 10, comprenant en outre l'étape suivante :
chauffage des particules séchées à au moins une température à laquelle on retire les constituants organiques encore présents.

12. Procédé selon la revendication 10 et 11, dans lequel les particules sont produites par un procédé aérosol, en particulier par un séchage par pulvérisation.

13. Procédé selon la revendication 10, dans lequel la dispersion, la suspension, le gel ou le sol apparaissent en outre avec utilisation de particules oxydes, dont le diamètre se situe en dessous de celui des particules ionomères à préparer, de préférence dans un intervalle de 1 à 10 %.

14. Procédé selon la revendication 10, comprenant en outre les étapes suivantes :
(o) production d'une dispersion de particules par hydrolyse effectuée avec précaution, le cas échéant par catalyse d'un acide ou une base, d'un oxyde métallique en solution alcoolique, séparation des particules obtenues à partir de la suspension, le cas échéant séchage des particules, le cas échéant chauffage des particules, pour séparer le matériau organique encore présent, et utilisation des particules pour l'obtention d'une dispersion, d'une suspension, du gel ou du sol selon (1) dans la revendication 10.

15. Procédé selon l'une des revendications 10 à 12, dans lequel les particules sont formées par production d'une émulsion, d'une dispersion ou d'une suspension, après quoi on les sépare du solvant qui les environne, ou bien on retire le solvant qui les entoure.

16. Procédé selon l'une des revendications 10 à 15,
dans lequel le ou les composants organiques est ou sont choisis parmi les alcoxysilanes, l'alcoolate d'aluminium et l'acylate de calcium.

17. Procédé selon la revendication 16,
dans lequel les composants organiques sont choisis parmi le butylate d'aluminium et l'acétate de calcium et dans lequel on utilise en outre le dioxyde de silicium.

18. Utilisation des particules ionomères sphériques ou presque sphériques selon l'une des revendications 1 à 9 dans les ciments dentaires, des ciments osseux ou des colles.

19. Utilisation de particules ionomères sphériques ou presque sphériques selon l'une des revendications 1 à 9 ou 18 avec des polyacides, en particulier des acides polyalcénoïques comme ciment.
